# EUROPEAN PATENT SPECIFICATION

(11) **EP 3 101 913 B1**
(45) Date of publication and mention of the grant of the patent: **04.07.2018**
(21) Application number: 15740502.8
(22) Date of filing: 21.01.2015
(51) Int. Cl.: B06B 1/06, H01L 41/277

(54) **STACKED ULTRASONIC VIBRATION DEVICE, PRODUCTION METHOD FOR STACKED ULTRASONIC VIBRATION DEVICE, AND ULTRASONIC MEDICAL APPARATUS**
GESTAPELTE ULTRASCHALLVIBRATIONSVORRICHTUNG, HERSTELLUNGSVERFAHREN FÜR DIE GESTAPELTE ULTRASCHALLVIBRATIONSVORRICHTUNG UND MEDIZINISCHE ULTRASCHALLVORRICHTUNG
DISPOSITIF À VIBRATIONS ULTRASONORES EMPILÉ, PROCÉDÉ DE PRODUCTION DE DISPOSITIF À VIBRATIONS ULTRASONORES EMPILÉ ET APPAREIL MÉDICAL ULTRASONORE

(30) Priority: 27.01.2014 JP 2014012686
(43) Date of publication of application: 07.12.2016
(73) Proprietor: Olympus Corporation, Tokyo 192-8507 (JP)
(72) Inventor: SHIOTANI, Koichi, Tokyo 192-8507 (JP); ITO, Hiroshi, Tokyo 192-8507 (JP)
(74) Representative: Gunzelmann, Rainer
(86) International application number: PCT/JP2015/051541
(87) International publication number: WO 2015/111621

(56) References cited:
- EP-A1- 2 881 183
- WO-A1-2014/020993
- JP-A- H04 343 282
- JP-A- 2004 350 349
- JP-A- 2008 128 875
- JP-A- 2008 128 875
- JP-A- 2008 227 123
- JP-A- 2013 135 301
- US-A- 4 582 240
- US-A1- 2008 212 408
- US-B1- 6 291 930

## Description

### Technical Field

The present invention relates to a stacked ultrasound vibration device that excites ultrasound vibration, a manufacturing method for the stacked ultrasound vibration device, and an ultrasound medical apparatus including the stacked ultrasound vibration device.

### Background Art

As an ultrasound treatment instrument that performs coagulation/dissection treatment of a biological tissue using ultrasound vibration, there is an ultrasound treatment instrument incorporating, in a hand piece, as an ultrasound vibration source, an ultrasound oscillator in which a piezoelectric transducer is used.

As such an ultrasound oscillator, there is an ultrasound oscillator in which a piezoelectric element, which converts an electric signal into mechanical vibration, is sandwiched by two block-like metal members functioning as a front mass or a back mass and integrated by some method including bonding to vibrate. Such an ultrasound transducer is called Langevin transducer.

As the Langevin transducer, there is known a bolt-clamped Langevin-type transducer in which, for example, a piezoelectric element is sandwiched by two metal members and firmly fastened by a bolt as a method of integrating the piezoelectric element and the metal members and the entire piezoelectric element and metal members integrally vibrate.

In the piezoelectric element used in such a bolt-clamped Langevin-type transducer, in general, lead zirconate titanate (PZT, Pb(Zrₓ,Ti₁₋ₓ)O3) is used. A shape of the piezoelectric element is machined into a ring shape and a bolt is inserted through the piezoelectric element.

The PZT has high productivity and high electromechanical conversion efficiency and has a good characteristic as a piezoelectric material. Therefore, the PZT has been used in various fields of an ultrasound transducer, an actuator, and the like for long years.

However, since lead is used in the PZT, from a viewpoint of an adverse effect to environment, in recent years, there has been a demand for use of a non-lead piezoelectric material in which lead is not used. As such a non-lead piezoelectric material having high electromechanical conversion efficiency, lithium niobate (LiNbO3) of a piezoelectric single crystal is known.

As a configuration for inexpensively realizing a Langevin transducer in which lithium niobate is used, there has been known a method of integrally bonding a piezoelectric element and metal blocks in a state in which the piezoelectric element is sandwiched by the metal blocks. In particular, when the metal blocks and the piezoelectric element are bonded by a brazing material such as solder without using an adhesive as a method for bonding the metal blocks and the piezoelectric element, a vibration characteristic more satisfactory than that of the adhesive is obtained in the Langevin transducer.

However, when the metal blocks and the piezoelectric element are bonded by the brazing material such as solder, in general, a high-temperature process is necessary. There is a problem in that the piezoelectric element of the piezoelectric single crystal is cracked by thermal stress in a different-material bonded section, which is a portion where the metal blocks and the piezoelectric element are bonded.

As a technique for solving such a problem, an ultrasound oscillator of Japanese Patent Application Laid-Open Publication No. 2008-128875 is disclosed. In the ultrasound oscillator in the past, there is known a technique for providing structures such as grooves or hollows on bonding surfaces of respective metal blocks bonded by an adhesive to electrodes provided on upper and lower both surfaces of a piezoelectric transducer to achieve suppression of occurrence of a shearing strain that occurs during driving, a reduction in a dielectric loss on the bonding surfaces, prevent occurrence of a crack in the piezoelectric transducer, and stabilize a vibration mode.

However, in the ultrasound oscillator in the past disclosed in Japanese Patent Application Laid-Open Publication No. 2008-128875, there is a problem in that a machining process is necessary for the metal block surfaces and manufacturing costs are increased.

US patents US4582240 and US6291930 also consider bonding of piezoelectric materials at temperatures much lower than the Curie temperature of piezoelectric material.

That is, in the ultrasound oscillator in the past, the structures such as grooves or hollows are provided on the metal block surfaces in order to absorb thermal stress that occurs in the bonded section between the different materials when the metal blocks and the piezoelectric element are bonded by bonding, stress that occurs because of hardening and contraction of the adhesive, and the like. Therefore, there is a problem in that an excess machining process is necessary and costs increase.

In the ultrasound oscillator in the past, when a thermosetting adhesive is used for bonding and fixing of the piezoelectric transducer and the metal blocks, vicinities of bonding surfaces are heated when the adhesive is hardened. Consequently, in the ultrasound oscillator in the past, it is likely that a shearing strain equivalent to a temperature difference between a bonding temperature and a normal temperature occurs because of a difference between coefficients of thermal expansion of the piezoelectric transducer and the metal blocks.

Residual stress is always present on the bonding surfaces of the piezoelectric transducer and the metal blocks. There is also a problem in that a crack occurs on an inside of the piezoelectric transducer because of the residual stress.

Therefore, the present invention has been devised in view of the circumstances and an object of the present invention is to provide a stacked ultrasound vibration device that can be inexpensively manufactured and in which a piezoelectric body is prevented from being, for example, damaged by stress caused because of a difference between coefficients of thermal expansion of metal blocks functioning as mass materials and the piezoelectric body, a manufacturing method for the stacked ultrasound vibration device, and an ultrasound medical apparatus.

### Disclosure of Invention

### Means for Solving the Problem

A stacked ultrasound vibration device of an aspect in the present invention is a stacked ultrasound vibration device that comprises a plurality of piezoelectric bodies and mass material, the stacked ultrasound vibration device including: a stacked piezoelectric body unit in which the plurality of piezoelectric bodies and a plurality of electrode layers are stacked and integrated; a first bonding material that bonds the plurality of piezoelectric bodies and melts at a first bonding temperature lower than a half of a Curie temperature of the plurality of piezoelectric bodies; and a second bonding material that bonds the stacked piezoelectric body unit and the mass material and melts at a second bonding temperature lower than the first bonding temperature and higher than a maximum temperature during driving.

A manufacturing method for a stacked ultrasound vibration device of an aspect in the present invention is a manufacturing method for a stacked ultrasound vibration device including a stacked piezoelectric body unit in which the plurality of piezoelectric bodies and a plurality of electrode layers are stacked and integrated, a first bonding material that bonds the plurality of piezoelectric bodies and melts at a first bonding temperature lower than a half of a Curie temperature of the plurality of piezoelectric bodies, and a second bonding material that bonds the stacked piezoelectric body unit and mass material and melts at a second bonding temperature lower than the first bonding temperature and higher than a maximum temperature during driving, the manufacturing method including: a step in which a base metal is formed on front and rear surfaces of the plurality of piezoelectric single crystal wafers; a step of bonding the plurality of piezoelectric single crystal wafers, on which the base metal is formed, with the first bonding material to manufacture the stacked wafer; a step of dicing the stacked wafer to cut out the stacked piezoelectric body unit in plurality; and a step of bonding one of the stacked piezoelectric body unit with the mass material by the second bonding material to manufacture a stacked transducer.

Further, an ultrasound medical apparatus of an aspect in the present invention includes: a stacked ultrasound vibration device including a stacked piezoelectric body unit in which the plurality of piezoelectric bodies and a plurality of electrode layers are stacked and integrated, a first bonding material that bonds the plurality of piezoelectric bodies and melts at a first bonding temperature lower than a half of a Curie temperature of the plurality of piezoelectric bodies, and a second bonding material that bonds the stacked piezoelectric body unit and mass material and melts at a second bonding temperature lower than the first bonding temperature and higher than a maximum temperature during driving; and a probe distal end portion to which ultrasound vibration occurring in the stacked ultrasound vibration device is transmitted to treat a biological tissue.

According to the present invention described above, it is possible to provide a stacked ultrasound vibration device that can be inexpensively manufactured and in which a piezoelectric body is prevented from being, for example, damaged by stress caused because of a difference between coefficients of thermal expansion of metal blocks functioning as mass materials and the piezoelectric body, a manufacturing method for the stacked ultrasound vibration device, and an ultrasound medical apparatus.

### Brief Description of the Drawings

Fig. 1 is a sectional view showing an overall configuration of an ultrasound medical apparatus according to an aspect of the present invention;
Fig. 2 is a diagram showing a schematic configuration of an entire transducer unit according to the aspect of the present invention;
Fig. 3 is a perspective view showing a configuration of an ultrasound transducer according to the aspect of the present invention;
Fig. 4 is a side view showing the configuration of the ultrasound transducer according to the aspect of the present invention;
Fig. 5 is a flowchart showing a manufacturing process of the ultrasound transducer according to the aspect of the present invention;
Fig. 6 is a perspective view showing a piezoelectric single crystal wafer according to the aspect of the present invention;
Fig. 7 is a perspective view showing the piezoelectric single crystal wafer on which a base metal is formed according to the aspect of the present invention;
Fig. 8 is a perspective view showing a plurality of piezoelectric single crystal wafers to be stacked according to the aspect of the present invention;
Fig. 9 is a perspective view showing a stacked wafer in which the plurality of piezoelectric single crystal wafers are stacked according to the aspect of the present invention;
Fig. 10 is a perspective view showing the stacked wafer to be diced according to the aspect of the present invention;
Fig. 11 is a perspective view showing a stacked piezoelectric body unit cut out from the stacked wafer according to the aspect of the present invention;
Fig. 12 is an exploded perspective view of the transducer unit including the ultrasound transducer according to the aspect of the present invention;
Fig. 13 is an exploded perspective view showing a state in which a flexible printed board is mounted on the ultrasound transducer of the transducer unit according to the aspect of the present invention;
Fig. 14 is a perspective view showing the transducer unit in which the FPC is mounted on the ultrasound transducer according to the aspect of the present invention; and
Fig. 15 is a graph showing a temperature relation concerning manufacturing and driving of the ultrasound transducer according to the aspect of the present invention.

### Best Mode for Carrying Out the Invention

The present invention is explained below with reference to the figures. Note that, in the following explanation, it should be noted that the drawings based on respective embodiments are schematic and relations between thicknesses and widths of respective portions, ratios of the thicknesses of the respective portions, and the like are different from real ones. Portions having different relations and ratios of dimensions among the portions are sometimes included among the drawings.

First, an embodiment of an ultrasound medical apparatus including a stacked ultrasound vibration device that excites ultrasound vibration of an aspect of the present invention is explained below with reference to the drawings.

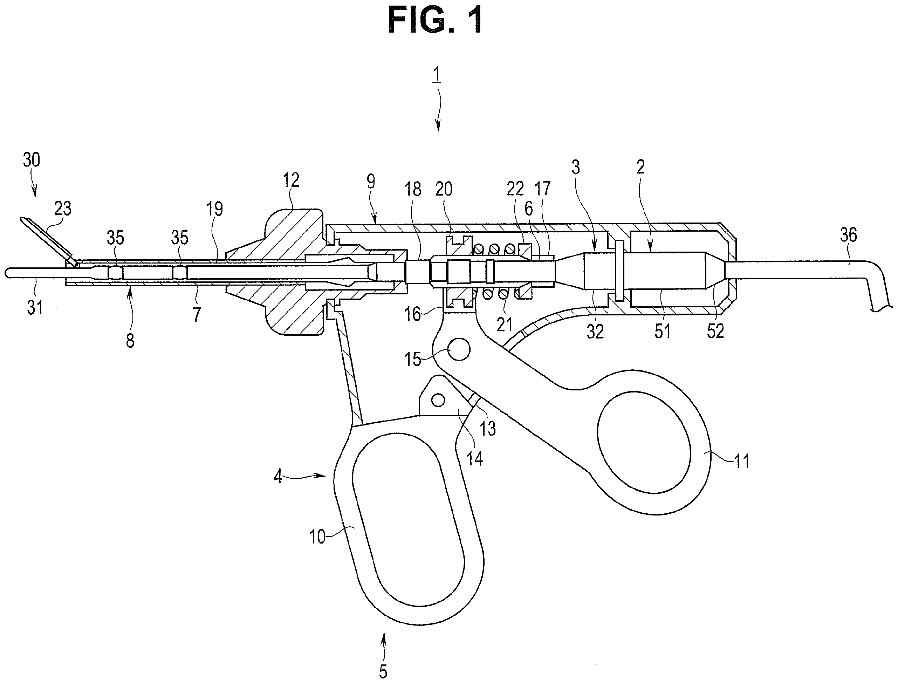

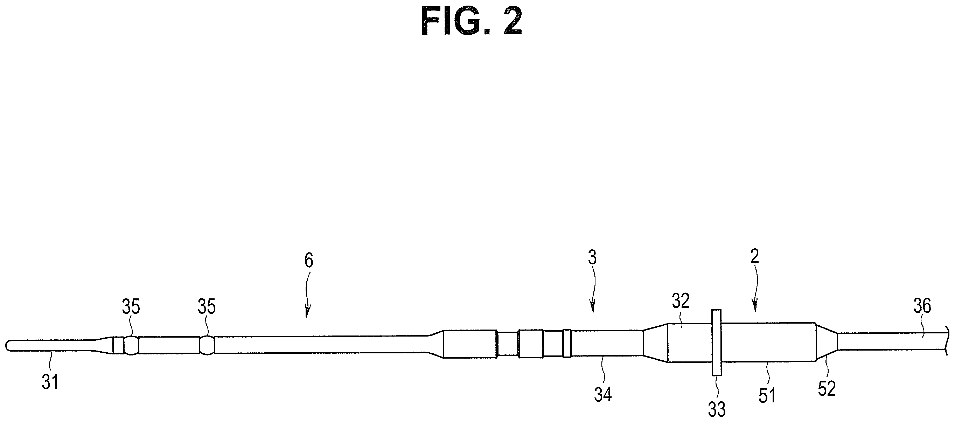

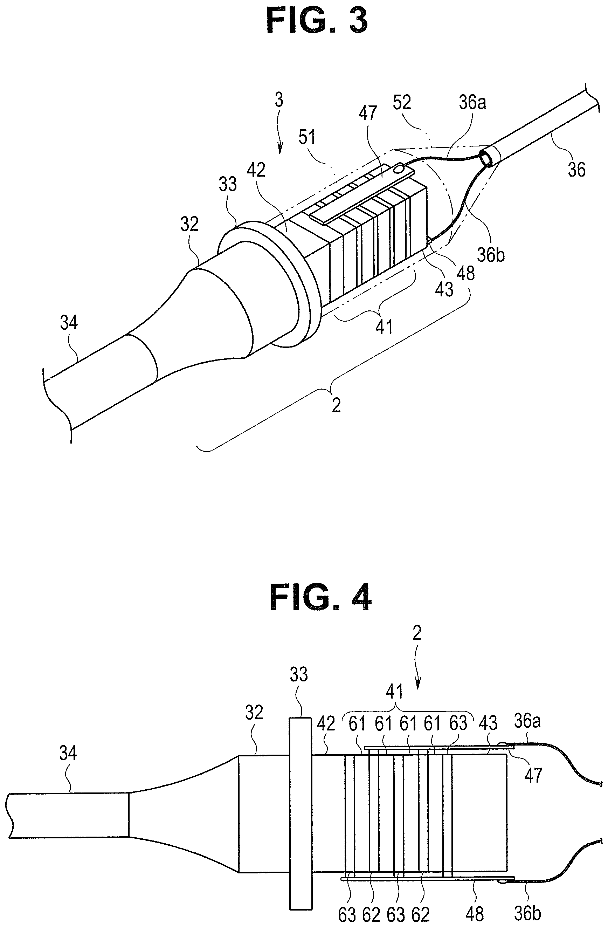

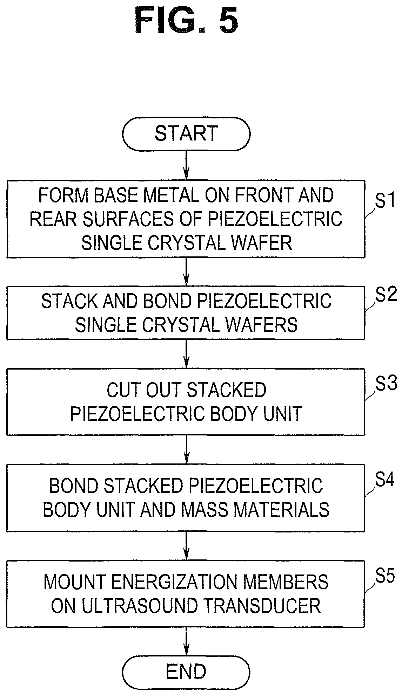

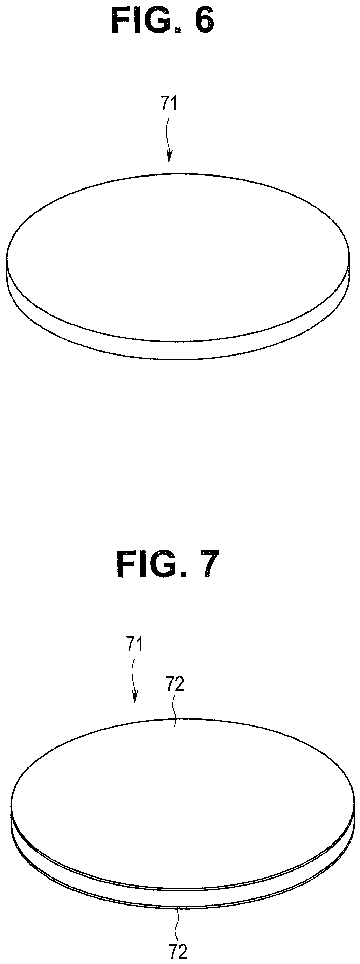

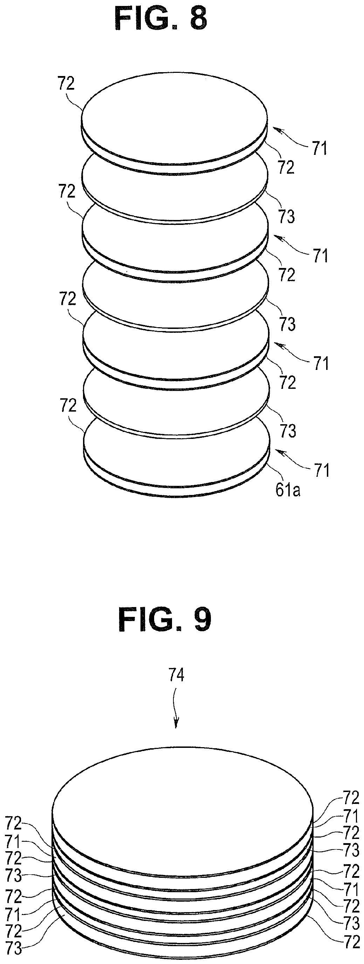

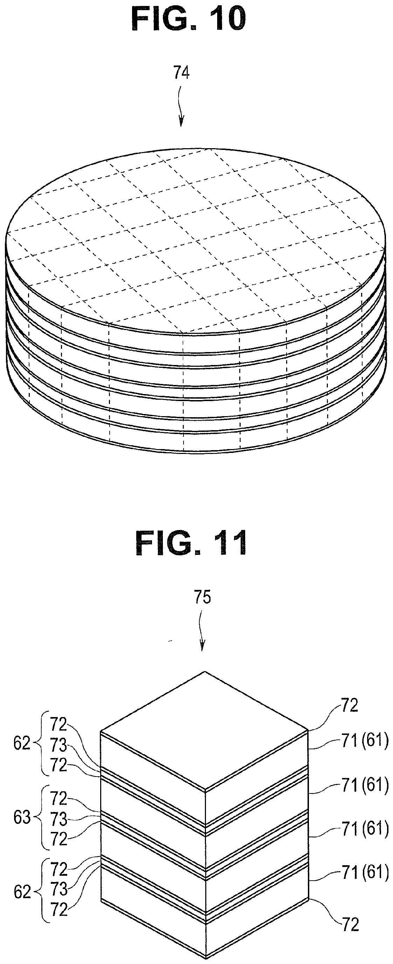

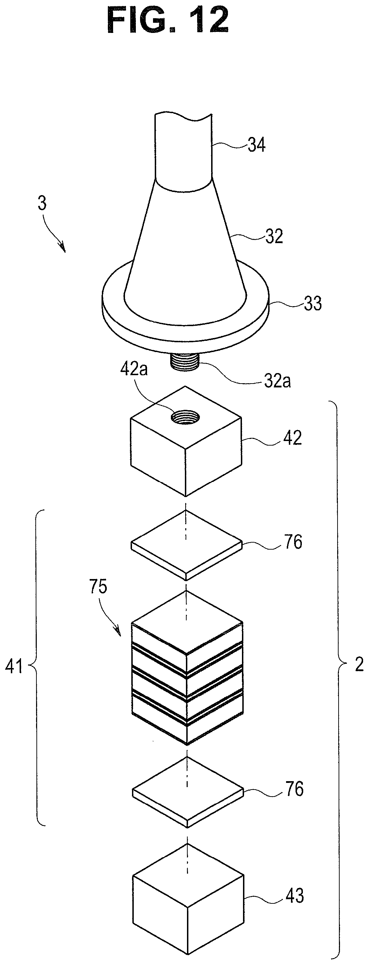

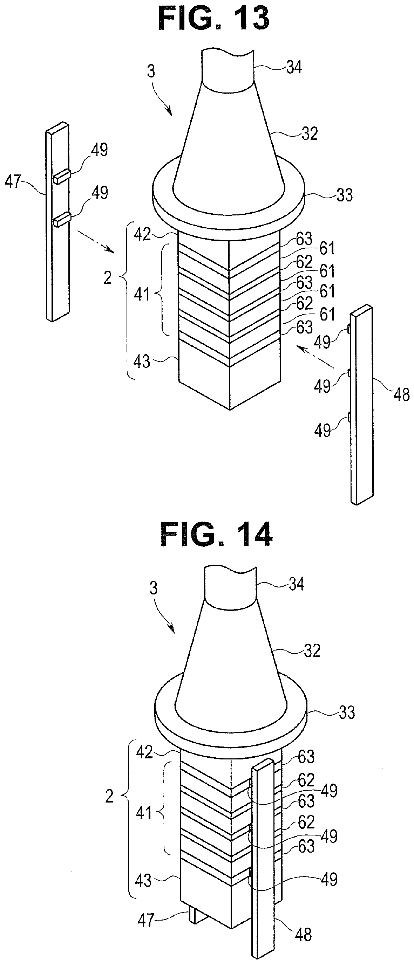

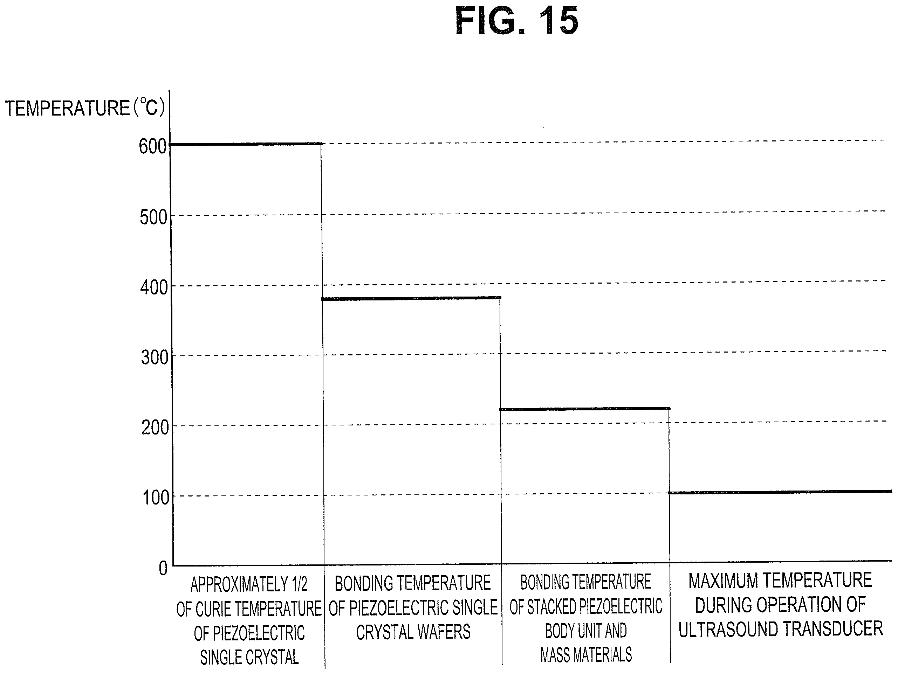

Fig. 1 is a sectional view showing an overall configuration of the ultrasound medical apparatus. Fig. 2 is a diagram showing a schematic configuration of an entire transducer unit. Fig. 3 is a perspective view showing a configuration of an ultrasound transducer. Fig. 4 is a side view showing the configuration of the ultrasound transducer. Fig. 5 is a flowchart showing a manufacturing process of the ultrasound transducer. Fig. 6 is a perspective view showing a piezoelectric single crystal wafer. Fig. 7 is a perspective view showing the piezoelectric single crystal wafer on which a base metal is formed. Fig. 8 is a perspective view showing a plurality of piezoelectric single crystal wafers to be stacked. Fig. 9 is a perspective view showing a stacked wafer in which the plurality of piezoelectric single crystal wafers are stacked. Fig. 10 is a perspective view showing the stacked wafer to be diced. Fig. 11 is a perspective view showing a stacked piezoelectric body unit cut out from the stacked wafer. Fig. 12 is an exploded perspective view of the transducer unit including the ultrasound transducer. Fig. 13 is an exploded perspective view showing a state in which an FPC is mounted on the ultrasound transducer of the transducer unit. Fig. 14 is a perspective view showing the transducer unit in which the FPC is mounted on the ultrasound transducer. Fig. 15 is a graph showing a temperature relation concerning manufacturing and driving of the ultrasound transducer.

### (Ultrasound medical apparatus)

An ultrasound medical apparatus 1 shown in Fig. 1 is provided with a transducer unit 3 including an ultrasound transducer 2 that mainly generates ultrasound vibration and a handle unit 4 that performs coagulation/dissection treatment of a diseased part using the ultrasound vibration.

The handle unit 4 includes an operation section 5, an insertion sheath section 8 including a long overcoat tube 7, and a distal end treatment section 30. A proximal end portion of the insertion sheath section 8 is attached to the operation section 5 to be capable of rotating in an axial direction.

The distal end treatment section 30 is provided at a distal end of the insertion sheath section 8. The operation section 5 of the handle unit 4 includes an operation section main body 9, a fixed handle 10, a movable handle 11, and a rotating knob 12. The operation section main body 9 is formed integrally with the fixed handle 10.

A slit 13, through which the movable handle 11 is inserted, is formed on a back side in a coupling section of the operation section main body 9 and the fixed handle 10. An upper part of the movable handle 11 is extended to an inside of the operation section main body 9 through the slit 13.

A handle stopper 14 is fixed to an end portion on a lower side of the slit 13. The movable handle 11 is rotatably attached to the operation section main body 9 via a handle support shaft 15. According to a motion of the movable handle 11 turning about the handle support shaft 15, the movable handle 11 is operated to be opened and closed with respect to the fixed handle 10.

A substantially U-shaped coupling arm 16 is provided at an upper end portion of the movable handle 11. The insertion sheath section 8 includes the overcoat tube 7 and an operation pipe 17 inserted through the overcoat tube 7 to be capable of moving in an axial direction.

A large diameter section 18 larger in diameter than a distal end side portion is formed on a proximal end portion of the overcoat tube 7. The rotating knob 12 is mounted around the large diameter section 18.

A ring-like slider 20 is provided on an outer circumferential surface of an operation pipe 19 to be capable of moving along the axial direction. A fixed ring 22 is disposed behind the slider 20 via a coil spring (an elastic member) 21.

Further, a proximal end portion of a grasping section 23 is coupled to a distal end portion of the operation pipe 19 via an action pin to be capable of turning. The grasping section 23 configures a treatment section of the ultrasound medical apparatus 1 in conjunction with a distal end portion 31 of a probe 6. During a motion of the operation pipe 19 moving in the axial direction, the grasping section 23 is operated to be pushed and pulled in a front-back direction via the action pin.

At this point, during a motion of the operation pipe 19 operated to be moved to a hand side, the grasping section 23 is turned in a counterclockwise direction about a fulcrum pin via the action pin.

Consequently, the grasping section 23 turns in a direction in which the grasping section 23 approaches the distal end portion 31 of the probe 6 (a closing direction). At this point, a biological tissue can be grasped between the grasping section 23 of a single swing type and the distal end portion 31 of the probe 6.

In a state in which the biological tissue is grasped in this way, electric power is supplied to the ultrasound transducer 2 from an ultrasound power supply to vibrate the ultrasound transducer 2. The ultrasound vibration is transmitted to the distal end portion 31 of the probe 6. Coagulation/dissection treatment of the biological tissue grasped between the grasping section 23 and the distal end portion 31 of the probe 6 is performed using the ultrasound vibration.

### (Transducer unit)

The transducer unit 3 is explained.

In the transducer unit 3, as shown in Fig. 2, the ultrasound transducer 2 and the probe 6, which is a bar-like vibration transmitting member that transmits ultrasound vibration occurring in the ultrasound transducer 2, are integrally assembled.

A horn 32, which amplifies amplitude of the ultrasound transducer, is concatenated to the ultrasound transducer 2. The horn 32 is made of stainless steel, duralumin, or a titanium alloy such as 64Ti (Ti-6Al-4V).

The horn 32 is formed in a conical shape, an outer diameter of which decreases toward a distal end side. An outward flange 33 is disposed in a proximal end outer circumferential section of the horn 32. Note that a shape of the horn 32 is not limited to the conical shape and may be, for example, an exponential shape, an outer diameter of which exponentially decreases toward a distal end side or a step shape that decreases in diameter stepwise toward a distal end side.

The probe 6 includes a probe main body 34 made of a titanium alloy such as 64Ti (Ti-6Al-4V). The ultrasound transducer 2 concatenated to the above-described horn 32 is disposed on a proximal end side of the probe main body 34.

In this way, the transducer unit 3 obtained by integrating the probe 6 and the ultrasound transducer 2 is formed. Note that the probe main body 34 and the horn 32 are screwed in the probe 6. The probe main body 34 and the horn 32 are bonded.

Ultrasound vibration generated in the ultrasound transducer 2 is amplified by the horn 32 and thereafter transmitted to the distal end portion 31 side of the probe 6. A treatment section explained below, which treats a biological tissue, is disposed at the distal end portion 31 of the probe 6.

On an outer circumferential surface of the probe main body 34, two rubber linings 35 formed in a ring shape by an elastic member are attached at an interval in several places of node positions of vibration halfway in the axial direction. Contact of the outer circumferential surface of the probe main body 34 and the operation pipe 19 explained below is prevented by the rubber linings 35.

That is, when the insertion sheath section 8 is assembled, the probe 6 functioning as a transducer-integrated probe is inserted into an inside of the operation pipe 19. At this point, the contact of the outer circumferential surface of the probe main body 34 and the operation pipe 19 is prevented by the rubber linings 35.

The ultrasound transducer 2 is electrically connected to, via an electric cable 36, a not-shown power supply device main body that supplies an electric current for generating ultrasound vibration. Electric power is supplied from the power supply device main body to the ultrasound transducer 2 through a wire in the electric cable 36, whereby the ultrasound transducer 2 is driven.

According to the above explanation, the transducer unit 3 includes the ultrasound transducer 2 that generates ultrasound vibration, the horn 32 that amplifies the ultrasound vibration generated by the ultrasound transducer 2, and the probe 6 that transmits the amplified ultrasound vibration.

### (Ultrasound transducer)

The ultrasound transducer 2 functioning as a stacked ultrasound vibration device of the present invention is explained below.

The ultrasound transducer 2 of the transducer unit 3 includes, as shown in Fig. 3 and Fig. 4, in order from a distal end, the above-described horn 32 connected to the probe main body 34, which is one of vibration transmitting members, by screwing or the like, a stacked transducer 41 functioning as a stacked piezoelectric body unit having a rectangular shape (a quadratic prim shape) concatenated behind the horn 32, and a cover body 51 that covers the stacked transducer 41 from a proximal end of the horn 32 to the electric cable 36.

Note that the cover body 51 covering the stacked transducer 41 includes, in a proximal end portion, a bending prevention piece 52 that covers wires 36a and 36b of the electric cable 36 electrically connected to two FPCs (flexible printed boards) 47 and 48 functioning as energizing members. Note that the energizing members are not limited to the FPCs 47 and 48 and may be a mere metal wire.

A front side of the stacked transducer 41 is bonded to a front mass 42 having a rectangular shape (a quadratic prism shape) connected to the horn 32 by screwing or the like and a rear side is connected to a back mass 43 having a rectangular shape (a quadratic prism shape).

Note that, like the horn 32, the front mass 42 and the back mass 43 are formed by duralumin. The front mass 42 and the back mass 43 may be formed by stainless steel or a titanium alloy such as 64Ti (Ti-6Al-4V).

Further, the stacked transducer 41 may hold, between the front mass 42 and the back mass 43, an insulating member that has insulation and in which vibration is less easily attenuated. As the insulating member, an insulation plate obtained by forming a ceramics-based material such as alumina or silicon nitride as a plate body having a rectangular shape (a quadratic prism shape).

In this way, in the stacked transducer 41, since the insulating member is provided, even when the ultrasound medical apparatus 1 shown in Fig. 1 is used together with a treatment instrument such as a medical high-frequency knife for performing treatment using a high frequency, damage or the like due to the high frequency from the treatment instrument is prevented.

As the stacked transducer 41, a piezoelectric element made of a non-lead single crystal material having a high Curie point is used. A plurality of, that is, four piezoelectric single crystal bodies 61 functioning as piezoelectric single crystal chips, which are piezoelectric elements, are stacked and disposed.

Among the four piezoelectric single crystal bodies 61, the front mass 42, and the back mass 43, positive side bonded metal 62 functioning as positive electrode layers and negative side bonded metal 63 functioning as negative electrode layers are alternately interposed as bonded metal layers formed from non-lead solder explained below functioning as a brazing material that bond the piezoelectric single crystal bodies 61, the front mass 42, and the back mass 43.

Note that, in the stacked transducer 41, electric contacts of the FPCs 47 and 48 are electrically connected to, by conductive paste or the like, the positive side bonded metal 62 or the negative side bonded metal 63 provided among the piezoelectric single crystal bodies 61 and between the piezoelectric single crystal bodies 61 and the front mass 42 or the back mass 43.

### (Manufacturing method for the ultrasound transducer)

A manufacturing method for the ultrasound transducer 2 explained above is explained in detail below.

First, the ultrasound transducer 2 is created from a piezoelectric single crystal wafer 71 (see Fig. 6) made of lithium niobate (LiNbO3) wafer functioning as a single crystal material using a piezoelectric material that has a high Curie temperature (Curie point) and a piezoelectric characteristic of which is not deteriorated even at a melting point of bonded metal.

Note that, as the piezoelectric single crystal wafer 71, a piezoelectric single crystal wafer having a crystal orientation called 36-degree rotation Y cut is used such that an electromechanical coupling coefficient in a thickness direction of the piezoelectric element increases.

As shown in the flowchart of Fig. 5, firstly, a base metal 72 (see Fig. 7) is formed on front and rear surfaces of the piezoelectric single crystal wafer 71 (see Fig. 6) (S1).

More specifically, in the piezoelectric single crystal wafer 71, the base metal 72 made of, for example, Ti/Ni/Au, Ti/Pt/Au, Cr/Ni/Au, or Cr/Ni/Pd/Au having satisfactory adhesion and wettability with non-lead solder is formed on the front and rear surfaces by vapor deposition, sputtering, plating, or the like.

Subsequently, the piezoelectric single crystal wafers 71 as many as a number corresponding to desired specifications of the ultrasound transducer 2, that is, four piezoelectric single crystal wafers 71 have (see Fig. 8 and Fig. 9) are stacked and bonded (S2).

More specifically, among the base metal 72 of the four piezoelectric single crystal wafers 71, as shown in Fig. 8, first brazing materials 73 functioning as a first bonding material, in which non-lead solder, for example, Zn-Al-based solder is used, are provided. Note that the first brazing material 73 is disposed on one base metal 72 of the piezoelectric single crystal wafer 71 by screen printing or a ribbon form.

Since the Zn-Al-based solder is used in the first brazing materials 73 that bond the four piezoelectric single crystal wafers 71 one another, the four piezoelectric single crystal wafers 71 are heated to approximately 380°C, which is a first bonding temperature at which the first brazing materials 73 melt, and slowly cooled. In this way, as shown in Fig. 9, the four piezoelectric single crystal wafers 71 are bonded to one another by the first brazing materials 73.

Consequently, bonded metal layers configured by the base metal 72 and the first brazing materials 73 are formed among the four piezoelectric single crystal wafers 71. An integrated stacked wafer 74 is formed.

Note that, in a heating process for bonding the four piezoelectric single crystal wafers 71, pressurization is desirably performed to compress the four piezoelectric single crystal wafers 71 in a stacking direction according to necessity.

Subsequently, a stacked piezoelectric body unit 75 (see Fig. 10 and Fig. 11) is cut out from the stacked wafer 74 (S3).

More specifically, the stacked wafer 74 is cut out in a block shape along a broken line (an imaginary line) shown in Fig. 10 by a thin dicing blade. A plurality of stacked piezoelectric body unit 75 functioning as rectangular piezoelectric material blocks shown in Fig. 11 are extracted.

That is, the stacked piezoelectric body unit 75 integrated in a state in which the four piezoelectric single crystal bodies 61 are stacked is extracted from the stacked wafer 74. The base metal 72 and the first brazing materials 73 among the four piezoelectric single crystal bodies 61 configure the positive side bonded metal 62 or the negative side bonded metal 63 of the stacked piezoelectric body unit 75.

A shape of the stacked piezoelectric body unit 75 is a rectangular block shape adjusted to specifications of the ultrasound transducer 2. Note that the stacked piezoelectric body unit 75 cut out by dicing from the stacked wafer 74 can be manufactured most inexpensively by forming the stacked piezoelectric body unit 75 in a rectangular block shape.

Subsequently, the cut out one stacked piezoelectric body unit 75 and the front mass 42 and the back mass 43, which are mass materials, are bonded (S4).

More specifically, as shown in Fig. 12, the front mass 42 and the back mass 43, which are metal blocks, are bonded such that both ends of the stacked piezoelectric body unit 75 are sandwiched.

Second brazing materials 76 functioning as second bonding materials, in which, for example, Sn-Ag-Cu-based solder is used as non-lead solder, are provided between the stacked piezoelectric body unit 75 and the front mass 42 and the back mass 43. The second brazing materials 76 are disposed on surfaces of the base metal 72 on both end faces of the stacked piezoelectric body unit 75 or respective one end faces of the front mass 42 and the back mass 43 by screen printing or a ribbon form.

Since the Sn-Ag-Cu-based solder is used in the second brazing materials 76 that bond the stacked piezoelectric body unit 75 and the front mass 42 and the back mass 43 each other, the stacked piezoelectric body unit 75 and the front mass 42 and the back mass 43 are heated to approximately 220°C, which is temperature at which the Sn-Ag-Cu-based solder melts, and slowly cooled. In this way, the stacked piezoelectric body unit 75 and the front mass 42 and the back mass 43 are bonded to each other by the second brazing materials 76.

Consequently, bonded metal layers configured by the base metal 72 and the second brazing materials 76 are formed between the stacked piezoelectric body unit 75 and the front mass 42 and the back mass 43. The integrated ultrasound transducer 2 is created. Note that, as explained above, in a heating process for bonding the stacked piezoelectric body unit 75 and the front mass 42 and the back mass 43, pressurization is desirably performed to compress the stacked piezoelectric body unit 75 and the front mass 42 and the back mass 43 in a stacking direction according to necessity.

In the ultrasound transducer 2, the second brazing material 76 interposed between the stacked piezoelectric body unit 75 and the front mass 42 or the back mass 43 and the base metal 72 provided on both the end faces of the stacked piezoelectric body unit 75 configure the negative side bonded metal 63 of the stacked piezoelectric body unit 75.

Note that, in a one end face center of the front mass 42, a screw hole 42a attached with a tap is machined. A screw section 32a integrally formed in the horn 32 is screwed in the screw hole 42a, whereby the horn 32 and the front mass 42 are screwed.

The two FPCs 47 and 48 (see Fig. 13 and Fig. 14), which are energization members, are mounted on the ultrasound transducer 2 (S5).

As shown in Fig. 13 and Fig. 14, the positive side bonded metal 62 and the negative side bonded metal 63 of the ultrasound transducer 2 are electrically connected to the electric contacts of the FPCs 47 and 48 via electric connection sections 49 formed using conductive paste or the like.

That is, in order to take electric connection of the positive side bonded metal 62 and the negative side bonded metal 63 and the FPCs 47 and 48, the electric contacts of the FPCs 47 and 48 are set in contact with outer surfaces of the positive side bonded metal 62 and the negative side bonded metal 63 via the electric connection sections 49. The FPCs 47 and 48 are fixed to the stacked piezoelectric body unit 75.

In this way, electric connection of the positive side bonded metal 62 and the negative side bonded metal 63 and the FPCs 47 and 48 is established. The wires 36a and 36b (see Fig. 3 and Fig. 4) of the above-described electric cable 36 are connected to the FPCs 47 and 48.

Note that, in Fig. 13 and Fig. 14, a state is shown in which the horn 32 and the front mass 42 are screwed. However, bonding by the screwing of the horn 32 and the front mass 42 only has to be performed before or after the mounting of the FPCs 47 and 48 on the ultrasound transducer 2 in step S5 explained above.

With such a configuration, as a positive electrode side, the wire 36a of the electric cable 36, the FPC 47, the electric connection sections 49, and the positive electrode side metal 62 are electrically connected. As a negative electrode side, the wire 36b of the electric cable 36, the FPC 48, the electric connection sections 49, and the negative electrode metal 63 are electrically connected. According to the electric connections, a driving signal is applied to the four piezoelectric single crystal bodies 61 via the positive side bonded metal 62 and fed back from the negative side bonded metal 63.

Note that exposed surface portions of the positive side bonded metal 62, the negative side bonded metal 63, and the electric connection sections 49 may be covered with an insulator such as resin to prevent occurrence of unnecessary electric connection that leads to a failure. For the purpose of reinforcement of mechanical fixing of the FPCs 47 and 48, the FPCs 47 and 48 may be fixed to the positive side bonded metal 62 and the negative side bonded metal 63 by an adhesive. Further, the FPCs 47 and 48 may be fixed to surfaces of side portions of the four piezoelectric single crystal bodies 61 by the adhesive.

According to the manufacturing process of the ultrasound transducer 2 explained above, the front mass 42, the four piezoelectric single crystal bodies 61, and the back mass 43 are stacked and integrated by the positive side bonded metal 62 and the negative side bonded metal 63 functioning as the bonded metal layers. The driving signal is applied to, via the electric connection sections 49, the positive side bonded metal 62 from the FPCs 47 and 48 provided on the side surfaces of the stacked body and fed back by the negative side bonded metal 63 to ultrasonically vibrate the entire ultrasound transducer 2.

In this way, in the ultrasound medical apparatus 1 of the present embodiment, the ultrasound transducer 2 functioning as the stacked ultrasound vibration device is configured to include the rectangular block-like stacked piezoelectric body unit 75, in which the four piezoelectric single crystal bodies 61, the positive side bonded metal 62, and the negative side bonded metal 63 are stacked, by forming the base metal 72 on the plurality of, that is, four piezoelectric single crystal wafers 71 and thereafter dicing and cutting out the stacked wafer 74 obtained by stacking and bonding the four piezoelectric single crystal wafers 71 by the first brazing materials 73.

Consequently, a process for individually bonding the piezoelectric single crystal bodies 61 is unnecessary in the stacked piezoelectric body unit 75 provided in the ultrasound transducer 2. It is possible to collectively manufacture a plurality of stacked piezoelectric body units 75 from the stacked wafer 74.

Therefore, in the ultrasound transducer 2, since it is unnecessary to individually cut out the piezoelectric single crystal bodies 61 from the piezoelectric single crystal wafer 71, the number of times of dicing decreases and a machining time is reduced, leading to a reduction in costs. As a result, it is possible to inexpensively manufacture the ultrasound transducer 2.

Note that, in the above explanation, the example of the rectangular block-like shape of the ultrasound transducer 2 that can be most inexpensively manufactured is explained. However, the shape of the ultrasound transducer 2 is not limited to this. The shape of these members may be, for example, columnar shape. Further, there is no limitation either in cutting out the stacked piezoelectric body unit 75 from the stacked wafer 74 with dicing and forming the stacked piezoelectric body unit 75 in the rectangular block-like shape. For example, it is also possible that the piezoelectric single crystal wafer 71, on which the base metal 72 is formed, is diced into a rectangular shape in advance and the stacked and bonded stacked piezoelectric body unit 75 is formed using a plurality of piezoelectric single crystal wafers 71.

In the ultrasound transducer 2, the Zn-Al-based solder is used in the first brazing materials 73 that bond the stacked four piezoelectric single crystal wafers 71. For the second brazing materials 76 that bond the stacked piezoelectric body unit 75, which is cut out from the stacked wafer 74, and the front mass 42 and the back mass 43, the Sn-Ag-Cu-based solder having the melting point (the melting temperature) lower than the melting point of the first brazing materials 73 is used.

More specifically, the first bonding temperature in bonding the four piezoelectric single crystal wafers 71 is set to a melting temperature (approximately 380°) of the first brazing materials 73 and the second bonding temperature in bonding the stacked piezoelectric body unit 75, which is cut out from the stacked wafer 74, and the front mass 42 and the back mass 43 is set to a melting temperature (approximately 220°C) of the second brazing materials 76 lower than the first bonding temperature.

That is, in the ultrasound transducer 2, as shown in Fig. 15, the first bonding temperature for bonding the four piezoelectric single crystal wafers 71 (the piezoelectric single crystal bodies 61) is approximately 380°C and the second bonding temperature for bonding the stacked piezoelectric body unit 75, which is cut out from the stacked wafer 74, and the front mass 42 and the back mass 43 is approximately 220°C lower than the first bonding temperature (approximately 380°C).

When the stacked piezoelectric body unit 75 is bonded to the front mass 42 and the back mass 43, the four piezoelectric single crystal bodies 61 are bonded by the first brazing materials 73. Even if the four piezoelectric single crystal bodies 61 are heated to the melting temperature (approximately 220°C) of the second brazing materials 76, since the first brazing materials 73 having the melting temperature (approximately 380°C) higher than the melting temperature of the second brazing materials 76 is used, the first brazing materials 73 that bond the piezoelectric single crystal bodies 61 do not melt. Reliability of the bonding among the piezoelectric single crystal bodies 61 is not spoiled.

Incidentally, in the manufacturing process of the ultrasound transducer 2, when the front mass 42 and the back mass 43 are bonded to the stacked piezoelectric body unit 75, in some case, the piezoelectric single crystal bodies 61 are affected and a crack occurs on insides of the piezoelectric single crystal bodies 61. This is because, in the ultrasound transducer 2, according to a difference between coefficients of thermal expansion of the piezoelectric single crystal bodies 61 and the front mass 42 and the back mass 43, in some case, a shearing strain occurs on the piezoelectric single crystal bodies 61 side and residual stress always occurs.

As explained above, the melting temperature of the second brazing materials 76 used in bonding parts of the front mass 42 or the back mass 43 and the stacked piezoelectric body unit 7 is set lower than the melting temperature of the first brazing materials 73. Consequently, it is possible to suppress the influence due to the difference between the coefficients of thermal expansion. It is possible to prevent a crack that occurs on the insides of the piezoelectric single crystal bodies 61.

Further, in the ultrasound transducer 2, lithium niobate (LiNbO3) is used in the four piezoelectric single crystal bodies 61 stacked as the stacked piezoelectric body unit 75. A Curie point (a Curie temperature) of the lithium niobate (LiNbO3) is approximately 1200°C. Approximately 600°C, which is a half temperature of the Curie point, is considered to be an upper limit of usability.

In the ultrasound transducer 2, as a maximum temperature during manufacturing, the first bonding temperature for melting the first brazing materials 73 when the four piezoelectric single crystal wafers 71 are bonded and bonding the four piezoelectric single crystal wafers 71 (the piezoelectric single crystal bodies 61) is approximately 380°C lower than approximately 600°C, which is a half of the Curie temperature of the piezoelectric single crystal bodies 61. Therefore, piezoelectric performance of the four piezoelectric single crystal bodies 61 formed from the piezoelectric single crystal wafer 71 is not deteriorated.

Incidentally, in the ultrasound transducer 2, temperature during operation rises to a maximum temperature of approximately 100°C. That is, in the ultrasound transducer 2, as shown in Fig. 15, the melting temperature (approximately 380°C) of the first brazing materials 73 that bond the four piezoelectric single crystal wafers 71 and the melting temperature (approximately 220°C) of the second brazing materials 76 that bond the stacked piezoelectric body unit 75 and the front mass 42 and the back mass 43 are higher than the maximum temperature (approximately 100°C) during the operation.

Therefore, during the operation, temperature of the ultrasound transducer 2 does not reach the temperature at which the positive side bonded metal 62 and the negative side bonded metal 63 formed from the first brazing materials 73 and the second brazing materials 76 melt. Therefore, reliability of the bonding among the four piezoelectric single crystal bodies 61 and the bonding of the stacked piezoelectric body unit 75 and the front mass 42 and the back mass 43 are not spoiled.

In other words, in the ultrasound transducer 2, the second bonding temperature (approximately 220°C) for bonding the stacked piezoelectric body unit 75 and the front mass 42 and the back mass 43 is temperature lower than the first bonding temperature (approximately 380°C) at the time when the four piezoelectric single crystal wafers 71 are bonded, which is the manufacturing time of the stacked piezoelectric body unit 75, and higher than the maximum temperature (approximately 100°C) during the operation of the ultrasound transducer 2.

As explained above, the ultrasound transducer 2 is set in a temperature relation in which the second bonding temperature (approximately 220°C) is set between the maximum temperature (approximately 100°C) during the driving and the first bonding temperature (approximately 380°C). Therefore, it is possible to manufacture the ultrasound transducer 2 without spoiling the reliability of the bonding among the four piezoelectric single crystal bodies 61 and the bonding of the stacked piezoelectric body unit 75 and the front mass 42 and the back mass 43.

In addition, the ultrasound transducer 2 is configured such that the positive side bonded metal 62 and the negative side bonded metal 63, which are bonded sections among the four piezoelectric single crystal bodies 61 and bonded sections between the stacked piezoelectric body unit 75 and the front mass 42 and the back mass 43, are not affected even if the temperature of the ultrasound transducer 2 reaches the maximum temperature of approximately 100°C during the operation.

In the ultrasound transducer 2 of the present embodiment, a coefficient of thermal expansion of the second brazing materials 76, which are bonding materials of the parts where the stacked piezoelectric body unit 75 and the front mass 42 and the back mass 43 are bonded, is set in an intermediate range of coefficients of thermal expansion of the piezoelectric single crystal bodies 61 and the front mass 42 and the back mass 43.

Therefore, in the ultrasound transducer 2, it is possible to suppress a shearing strain on the piezoelectric single crystal bodies 61 side, which sometimes occur because of a difference between the coefficients of thermal expansion of the piezoelectric single crystal bodies 61 provided at both ends of the stacked piezoelectric body unit 75 and the front mass 42 and the back mass 43. It is possible to prevent a crack that occurs on the insides of the piezoelectric single crystal bodies 61.

More specifically, in the ultrasound transducer 2, lithium niobate (LiNbO3) is used in the piezoelectric single crystal bodies 61 and duralumin is used in the front mass 42 and the back mass 43. Note that a coefficient of thermal expansion of the lithium niobate (LiNbO3) is 8 to 15×10⁻⁶ [1/°C] and a coefficient of thermal expansion of the duralumin is 24×10⁻⁶ [1/°C].

Therefore, in the present embodiment, the second brazing materials 76, which are the Sn-Ag-Cu-based solder having a coefficient of thermal expansion between the coefficients of thermal expansion of the piezoelectric single crystal bodies 61 of the lithium niobate (LiNbO3) and the front mass 42 and the back mass 43 of the duralumin are used.

Note that the coefficient of thermal expansion of the Sn-Ag-Cu-based solder is 21×10⁻⁶ [1/°C] larger than the coefficient of thermal expansion (8 to 15×10⁻⁶ [1/°C]) of the lithium niobate (LiNbO3) and smaller than the coefficient of thermal expansion (24×10⁻⁶ [1/°C]) of the duralumin.

Consequently, the second brazing materials 76 of the Sn-Ag-Cu-based solder, which is the bonding material of the stacked piezoelectric body unit 75 and the front mass 42 and the back mass 43, play a role of absorbing a coefficient of thermal expansion difference between the stacked piezoelectric body unit 75 and the front mass 42 and the back mass 43. Stress to the piezoelectric single crystal bodies 61 is reduced. Occurrence of a crack on the insides of the piezoelectric single crystal bodies 61 is prevented.

Note that the second brazing materials 76 only have to have the coefficient of thermal expansion between the coefficients of thermal expansion of the lithium niobate (LiNbO3) and the duralumin. The second brazing materials 76 may be, for example, Sn-based solder, Sn-Ag-based solder, or Sn-Cu-based solder besides the Su-Ag-Cu-based solder.

The Zn-Al-based solder is used in the first brazing materials 73, which are the bonding materials of the piezoelectric single crystal bodies 61. However, other brazing materials may be used as long as a magnitude relation between the melting temperature of the first brazing materials 73 (the first bonding temperature) and the melting temperature of the second brazing materials 76 (the second bonding temperature) holds. That is, the first brazing materials 73 only have to have a melting temperature higher than the melting temperature of the second brazing materials 76 (the first bonding temperature > the second bonding temperature). However, a difference between the melting temperatures is desirably equal to or larger than several tens °C taking into account for temperature fluctuation during manufacturing.

Further, even in a state in which temperature of the solder used in the first brazing materials 73 and the second brazing materials 76 does not reach the melting temperature and the solder does not melt, a Young's modulus decreases and the solder softens when the temperature is near the melting temperature. Performance of the ultrasound transducer 2 is deteriorated. Therefore, in particular, it is desirable to use solder having a large difference between the melting temperature of the second brazing materials 76 and the maximum temperature (approximately 100°C) during the driving of the ultrasound transducer 2.

Incidentally, lead zirconate titanate (PZT, Pb(Zrₓ,Ti₁₋ₓ)O3) currently widely used as a piezoelectric material of an ultrasound transducer in the past has a Curie temperature of approximately 300°C. Approximately 150°C, which is a half of the Curie temperature, is considered to be an upper limit of usability. Therefore, even if low-melting point solder is used, a difference between the first bonding temperature and the second bonding temperature is not obtained. The low-melting point solder cannot be applied to the manufacturing method of the ultrasound transducer 2 in the present embodiment.

As explained above, the ultrasound transducer 2, which is the stacked ultrasound vibration device, and the ultrasound medical apparatus 1 including the ultrasound transducer 2 of the present embodiment can be inexpensively manufactured and is configured to be capable of preventing a piezoelectric body from being broken by stress that occurs because of a difference between coefficients of thermal expansion of the metal blocks functioning as the mass materials and the piezoelectric body.

This application is based upon and claims priority from Japanese Patent Application No. 2014-012686 filed in Japan on January 27, 2014.

## Claims

1. A stacked ultrasound vibration device that comprises a plurality of piezoelectric bodies (61) and mass material (42, 43), the stacked ultrasound vibration device comprising:
a stacked piezoelectric body unit (41) in which the plurality of piezoelectric bodies (61) and a plurality of electrode layers (62, 63) are stacked and integrated;
a first bonding material (73) configured to bond the plurality of piezoelectric bodies (61) and configured to melt at a first bonding temperature lower than a half of a Curie temperature of the plurality of piezoelectric bodies; and
a second bonding material (76) configured to bond the stacked piezoelectric body unit (41) and the mass material (42, 43) and configured to melt at a second bonding temperature lower than the first bonding temperature and higher than a maximum temperature during driving.

2. The stacked ultrasound vibration device according to claim 1, wherein a coefficient of thermal expansion of the second bonding material is in an intermediate range between a coefficient of thermal expansion of the plurality of piezoelectric bodies and a coefficient of thermal expansion of the mass material.

3. The stacked ultrasound vibration device according to claim 1 or 2, wherein the first bonding material and the second bonding material are different brazing materials.

4. The stacked ultrasound vibration device according to any one of claims 1 to 3, wherein the stacked piezoelectric body unit has a rectangular block shape.

5. The stacked ultrasound vibration device according to any one of claims 1 to 4, wherein the plurality of piezoelectric bodies are piezoelectric single crystal materials.

6. The stacked ultrasound vibration device according to any one of claims 1 to 5, wherein the first bonding material and the second bonding material are configured to function as the plurality of electrode layers.

7. A manufacturing method for the stacked ultrasound vibration device according to any one of claims 1 to 6, comprising:
a step (S1) in which a base metal is formed on front and rear surfaces of the plurality of piezoelectric single crystal wafers;
a step (S2) of bonding the plurality of piezoelectric single crystal wafers, on which the base metal is formed, with the first bonding material to create the stacked wafer;
a step (S3) of dicing the stacked wafer to cut out the stacked piezoelectric body unit in plurality; and
a step (S4) of bonding one of the stacked piezoelectric body unit with the mass material by the second bonding material to manufacture a stacked transducer.

8. The manufacturing method for the stacked ultrasound vibration device according to claim 7, wherein, after the first bonding material is heated to the first bonding temperature and melted, the first bonding material is cooled to bond the plurality of piezoelectric single crystal wafers.

9. The manufacturing method for the stacked ultrasound vibration device according to claim 7 or 8, wherein, after the second bonding material is heated to the second bonding temperature and melted, the second bonding material is cooled to bond the stacked piezoelectric body unit with the mass material.

10. An ultrasound medical apparatus (1) comprising:
the stacked ultrasound vibration device according to any one of claims 1 to 6; and
a probe distal end portion (30) to which ultrasound vibration occurring in the stacked ultrasound vibration device is transmitted to treat a biological tissue.

## Patentansprüche

1. Gestapelte Ultraschallvibrationsvorrichtung, welche eine Vielzahl von piezoelektrischen Körpern (61) und Massenmaterial (42, 43) umfasst,
die gestapelte Ultraschallvibrationsvorrichtung umfassend:
eine gestapelte piezoelektrische Körpereinheit (41), in welcher die Vielzahl von piezoelektrischen Körpern (61) und eine Vielzahl von Elektrodenschichten (62, 63) gestapelt und integriert sind;
ein erstes Bindungsmaterial (73), welches konfiguriert ist, um die Vielzahl von piezoelektrischen Körpern (61) zu binden und konfiguriert ist, um bei einer ersten Bindungstemperatur, welche niedriger als ein halb einer Curie-Temperatur der Mehrzahl von piezoelektrischen Körpern ist, zu schmelzen; und
ein zweites Bindungsmaterial (76), welches konfiguriert ist, um die gestapelten piezoelektrischen Körpereinheiten (41) und das Massenmaterial (42, 43) zu binden und konfiguriert ist, um bei einer zweiten Bindungstemperatur, welche niedriger als die erste Bindungstemperatur und höher als eine maximale Temperatur bei Betrieb ist, zu schmelzen.

2. Gestapelte Ultraschallvibrationsvorrichtung gemäß Anspruch 1, bei der ein Wärmeausdehnungskoeffizient des zweiten Bindungsmaterials in einem Zwischenbereich zwischen einem Wärmeausdehnungskoeffizienten der Vielzahl von piezoelektrischen Körpern und einem Wärmeausdehnungskoeffizienten des Massenmaterials liegt.

3. Gestapelte Ultraschallvibrationsvorrichtung gemäß zu Anspruch 1 oder 2, bei der das erste Bindungsmaterial und das zweite Bindungsmaterial verschiedene Lötmaterialien sind.

4. Gestapelte Ultraschallvibrationsvorrichtung gemäß einem der Ansprüche 1 bis 3, bei der die gestapelte piezoelektrische Körpereinheit eine rechteckige Blockform aufweist.

5. Gestapelte Ultraschallvibrationsvorrichtung gemäß einem der Ansprüche 1 bis 4, bei der die Vielzahl von piezoelektrischen Körpern piezoelektrische Einkristallmaterialien sind.

6. Gestapelte Ultraschallvibrationsvorrichtung gemäß einem der Ansprüche 1 bis 5, bei der das erste Bindungsmaterial und das zweite Bindungsmaterial konfiguriert sind, um als die Vielzahl von Elektrodenschichten zu fingieren.

7. Herstellungsverfahren für die gestapelte Ultraschallvibrationsvorrichtung gemäß zu einem der Ansprüche 1 bis 6, umfassend:
einen Schritt (S1), in welchem ein Basismetall auf vorderen und hinteren Oberflächen der Vielzahl von piezoelektrischen Einkristallscheiben gebildet wird;
einen Schritt (S2) zum Binden der Vielzahl von piezoelektrischen Einkristallscheiben, auf welchen die Basismetalle gebildet sind, mit dem ersten Bindungsmaterial, um die gestapelte Scheibe zu erzeugen;
einen Schritt (S3) zum Würfeln der gestapelten Scheibe, um die gestapelte piezoelektrische Körpereinheit in Vielzahl auszuschneiden; und
einen Schritt (S4) zum Binden Eines der gestapelten piezoelektrischen Körpereinheiten mit dem Massenmaterial durch das zweite Bindungsmaterial, um einen gestapelten Wandler zu erzeugen.

8. Herstellungsverfahren für die gestapelte Ultraschallvibrationsvorrichtung gemäß Anspruch 7, bei dem, nachdem das erste Bindungsmaterial auf die erste Bindungstemperatur erhitzt wurde und geschmolzen ist, das erste Bindungsmaterial abgekühlt wird, um die Vielzahl von piezoelektrischen Einkristallscheiben zu binden.

9. Herstellungsverfahren für die gestapelte Ultraschallvibrationsvorrichtung gemäß Anspruch 7 oder 8, bei dem, nachdem das zweite Bindungsmaterial auf die zweite Bindungstemperatur erhitzt wurde und geschmolzen ist, das zweite Bindungsmaterial abgekühlt wird, um die gestapelte piezoelektrische Körpereinheit mit dem Massenmaterial zu binden.

10. Medizinische Ultraschallvorrichtung (1) umfassend:
die gestapelte Ultraschallvibrationsvorrichtung gemäß einem der Ansprüche 1 bis 6; und
einen distalen Sondenendabschnitt (30), zu welchem in der gestapelten Ultraschallvibrationsvorrichtung entstehende Ultraschallvibration übermittelt werden, um biologisches Gewebe zu behandeln.

## Revendications

1. Dispositif à vibrations ultrasonores empilé qui comprend une pluralité de corps piézoélectriques (61) et une matière de masse (42, 43), le dispositif à vibrations ultrasonores empilé comprenant :
une unité (41) de corps piézoélectriques empilés dans laquelle la pluralité de corps piézoélectriques (61) et une pluralité de couches d'électrodes (62, 63) sont empilées et intégrées ;
une première matière de liaison (73) configurée pour lier la pluralité de corps piézoélectriques (61) et configurée pour fondre à une première température de liaison inférieure à une moitié d'une température de Curie de la pluralité de corps piézoélectriques ; et
une deuxième matière de liaison (76) configurée pour lier l'unité (41) de corps piézoélectriques empilés et la matière de masse (42, 43) et configurée pour fondre à une deuxième température de liaison inférieure à la première température de liaison et supérieure à une température maximum lors du pilotage.

2. Dispositif à vibrations ultrasonores empilé selon la revendication 1, dans lequel un coefficient de dilatation thermique de la deuxième matière de liaison est dans une plage intermédiaire entre un coefficient de dilatation thermique de la pluralité de corps piézoélectriques et un coefficient de dilatation thermique de la matière de masse.

3. Dispositif à vibrations ultrasonores empilé selon la revendication 1 ou 2, dans lequel la première matière de liaison et la deuxième matière de liaison sont des matières de brasage différentes.

4. Dispositif à vibrations ultrasonores empilé selon l'une quelconque des revendications 1 à 3, dans lequel l'unité de corps piézoélectriques empilés a une forme de bloc rectangulaire.

5. Dispositif à vibrations ultrasonores empilé selon l'une quelconque des revendications 1 à 4, dans lequel la pluralité de corps piézoélectriques sont des matières monocristallines piézoélectriques.

6. Dispositif à vibrations ultrasonores empilé selon l'une quelconque des revendications 1 à 5, dans lequel la première matière de liaison et la deuxième matière de liaison sont configurées pour fonctionner comme la pluralité de couches d'électrodes.

7. Procédé de fabrication pour le dispositif à vibrations ultrasonores empilé selon l'une quelconque des revendications 1 à 6, comprenant :
une étape (S1) dans laquelle un métal de base est formé sur des surfaces avant et arrière de la pluralité de tranches monocristallines piézoélectriques ;
une étape (S2) de liaison de la pluralité de tranches monocristallines piézoélectriques, sur lesquelles le métal de base est formé, avec la première matière de liaison pour créer la tranche empilée ;
une étape (S3) de découpage en dés de la tranche empilée pour découper l'unité de corps piézoélectriques empilés en une pluralité ; et
une étape (S4) de liaison d'un de l'unité de corps piézoélectriques empilés avec la matière de masse par la deuxième matière de liaison pour fabriquer un transducteur empilé.

8. Procédé de fabrication pour le dispositif à vibrations ultrasonores empilé selon la revendication 7, dans lequel, après que la première matière de liaison a été chauffée à la première température de liaison et fondue, la première matière de liaison est refroidie pour lier la pluralité de tranches monocristallines piézoélectriques.

9. Procédé de fabrication pour le dispositif à vibrations ultrasonores empilé selon la revendication 7 ou 8, dans lequel, après que la deuxième matière de liaison a été chauffée à la deuxième température de liaison et fondue, la deuxième matière de liaison est refroidie pour lier l'unité de corps piézoélectriques empilés avec la matière de masse.

10. Appareil médical à ultrasons (1) comprenant :
le dispositif à vibrations ultrasonores empilé selon l'une quelconque des revendications 1 à 6 ; et
une partie d'extrémité distale de sonde (30) à laquelle une vibration ultrasonore survenant dans le dispositif à vibrations ultrasonores empilé est transmise pour traiter un tissu biologique.
